# EUROPEAN PATENT SPECIFICATION

(11) **EP 4 179 044 B1**
(45) Date of publication and mention of the grant of the patent: **15.05.2024**
(21) Application number: 20739622.7
(22) Date of filing: 09.07.2020
(51) Int. Cl.: C10G 9/36, C10G 9/24, C10G 15/08, C01B 3/24, C07C 2/76

(54) **METHOD FOR STEAM CRACKING**
VERFAHREN ZUM DAMPFKRACKEN
PROCÉDÉ DE VAPOCRAQUAGE

(43) Date of publication of application: 17.05.2023
(73) Proprietor: BASF Antwerpen N.V., 2040 Antwerpen (BE); Borealis AG, 1020 Vienna (AT); SABIC Global Technologies B.V., 4612 PX Bergen op Zoom (NL); TotalEnergies OneTech Belgium, 7181 Seneffe (BE); Basell Polyolefine GmbH, 50389 Wesseling (DE)
(72) Inventor: JORDENS, Marc, Jozef, Maria, 2560 Nijlen (BE); VAVIZOS, Nikolaos, 1150 Vienna (AT); VERMEIREN, Walter, Josephus, 3530 Houthalen (BE); LEINZ, Martin, 45133 Essen (DE); BRANDTS, Paulus, Maria, 6141 BM Limbricht (NL); APPELMAN, Eric, Simon, Petrus, 3311 LB Dordrecht (NL); POHL, Michael, 55276 Oppenheim (DE); BARREAU, Christian, 13330 La Barben (FR); BECKER, Jens, 50931 Koeln (DE)
(74) Representative: Algemeen Octrooi- en Merkenbureau B.V.
(86) International application number: PCT/EP2020/069323
(87) International publication number: WO 2022/008052

(56) References cited:
- EP-A1- 3 164 207
- EP-B1- 3 164 207
- CN-A- 106 520 176
- CN-Y- 2 832 817
- US-A- 5 219 530
- US-A1- 2006 116 543
- US-A1- 2016 296 904
- AMGHIZAR ISMAËL ET AL: "Sustainable innovations in steam cracking: CO 2 neutral olefin production", REACTION CHEMISTRY & ENGINEERING, [Online] vol. 5, no. 2, 4 February 2020 (2020-02-04), pages 239-257, XP055958696, DOI: 10.1039/C9RE00398C Retrieved from the Internet: URL:https://pubs.rsc.org/en/content/articl epdf/2020/re/c9re00398c> [retrieved on 2022-09-07]

## Description

### Introduction

The present application relates to a process for cracking a hydrocarbon feedstock, using to the largest extent electrically powered equipment where the power is obtained from renewable sources or low-carbon sources.

Global warming and climate change caused by human emissions of greenhouse gases have become a major concern to the public. Major policy initiatives such as the Paris Climate Agreement of 2017 and the European Green deal of 2019 are demanding actions to reduce on those emissions from all sectors in society, including the chemical industry.

The chemical industry is a major source of greenhouse gas emissions, via the generation of energy required for its conversions through combustion, via emission of co-products, or via end-of-life incineration of its products.

One particular large greenhouse gas emission source is concentrated in a few large-scale processes carried out at high temperatures (above 500 °C), which require external heating by combustion of fossil hydrocarbons. Examples are steam cracking (the production of light olefins from crude oil, distillates like naphtha, or gases like ethane, propane and butane), catalytic cracking (production of light components for fuel or feedstock use from heavy petroleum fractions), and methane steam reforming (making hydrogen and carbon monoxide or carbon dioxide from natural gas).

Conventional steam crackers are complex industrial facilities that can be divided into three main zones, each of which has several types of equipment with very specific functions: (i) the hot zone including: pyrolysis or cracking furnaces, quench exchanger and quench loop, the columns of the hot separation train (ii) the compression zone including: a cracked gas compressor, purification and separation columns, dryers and (iii) the cold zone including: the cold box, de-methaniser, fractionating columns of the cold separation train, the C₂ and C₃ converters, the gasoline hydrostabilization reactor.

Conventional steam cracking is carried out in tubular reactors in direct-fired heaters (furnaces). Various tube sizes and configurations can be used, such as coiled tube, U-tube, or straight tube layouts. Tube diameters range from 1 to 4 inches. Each furnace consists of a convection zone in which the waste heat is recovered and a radiant zone in which pyrolysis takes place.

In order to reduce or even eliminate the greenhouse gas emissions from those processes, the use of renewable or low-carbon energy, and electricity in particular, to achieve these high temperatures will be a major step forward. This is not unheard of: many examples are known of industrial heating with electricity, for instance the use of arc furnaces to melt scrap metal or to produce phosphorus, or for tracing of pipelines. However, the ready availability and the low cost of fossil fuels in the petrochemical industry (where they are often a side stream from certain conversions) have kept electrical heating technologies from widespread use in large-scale installations.

US 2006/116543 relates to amethod of steam cracking hydrocarbons, which method consists in heating a mixture of hydrocarbons and steam to a desired temperature that is high enough to crack the hydrocarbons and transform them into olefins, wherein the source of energy needed for heating the mixture is supplied essentially by cogeneration using combustion of a fuel to produce simultaneously both heat energy and mechanical work which is transformed into electricity by an alternator or an electricity generator, and the mixture is initially subjected to preheating using the heat energy supplied by the cogeneration, and is subsequently heated to the desired cracking temperature by means of electrical heating using the electricity supplied by the cogeneration.

CN 106 520 176 relates to a method for producing small molecular olefins from polyolefin plastics, by first cracking the polyolefin plastics, and then mixing the cracked liquid/waxy products and optional liquid petroleum hydrocarbons with water wherein the heating method of the mild cracking device is microwave heating and/or electric heating.

CN 2 832 817 relates to a thermal cracking reaction kettle, including a kettle body, with feed ports and discharge ports respectively provided on the left and right sides of the upper part of the kettle body, wherein far-infrared heaters are provided both inside and outside the heating kettle.

The recent emphasis on greenhouse gas reductions have triggered research into such electrical heating methods. US20150321912 of BASF describes processes using among others electricity as heat source. The `912 publication relates to a method of carrying out heat-consuming processes, wherein the total energy required averaged over a year for the heat-consuming process originates from at least two different energy sources, where one of the energy sources is an electric energy source whose power varies in the range from 0 to 100% of the total power required, and three different energy modes can individually provide the total power required for the heat-consuming process: (i) exclusively electric energy, (ii) a mixture of electric energy and at least one further nonelectric energy source or (iii) exclusively nonelectric energy, where the changeover time in which the change from one energy mode to another energy mode is completed is not more than 30 minutes.

Linde (US20170130887) relates to the use of resistive electrical heating for a range of high-temperature processes, including steam cracking. It describes a device for heating a fluid, including at least one electrically conductive pipeline for accommodating the fluid, and at least one voltage source connected to a respective pipeline. The voltage source is designed to generate an electric current in the connected pipeline which heats the pipeline in order to heat the fluid.

EP3249027 and EP3249028 patents envisage that at least one of the tubular reactors will be heated both by using combustion heat generated by burning at least one fuel and by using electrical heat generated by electrical energy.

US20180243711 (Haldor Topsoe) describes the use of inductive electrical heating for many high-temperature processes, e.g. steam methane reforming. It relates to a reactor system for carrying out an endothermic catalytic chemical reaction in a given temperature range upon bringing a reactant into contact with a catalyst material. The reactor system includes a reactor unit arranged to accommodate catalyst material including one or more ferromagnetic macroscopic supports susceptible for induction heating where the one or more ferromagnetic macroscopic supports are ferromagnetic at temperatures up to an upper limit of the given temperature range. The one or more ferromagnetic macroscopic supports are coated with an oxide, and the oxide is impregnated with catalytically active particles. The reactor system moreover includes an induction coil arranged to be powered by a power source supplying alternating current and being positioned so as to generate an alternating magnetic field within the reactor unit upon energization by the power source, whereby the catalyst material is heated to a temperature within the temperature range by the alternating magnetic field.

WO2016001476 (Coolbrook) describes a shock wave reactor for thermal cracking of hydrocarbon-containing feedstock, comprising a casing wherein a duct is formed with inlet and outlet; a rotor, the periphery of which contains an axial- flow blade cascade; wherein the casing substantially encloses the periphery of the rotor and a number of stationary vane cascades inside the duct, and further wherein the cascades are configured to direct feedstock containing process stream to repeatedly pass said cascades in accordance with helical trajectory while propagating within the duct between the inlet and outlet and to generate stationary shock-waves to heat the feedstock. The axial- flow rotor cascade is configured to provide kinetic energy and to add velocity to the feedstock containing process stream, and the stationary vanes located downstream the rotor cascade are configured to reduce the velocity of the stream and convert kinetic energy into heat. The reactor may be configured for the realization of thermal cracking processes utilizing hydrocarbons.

Whereas all this documentation is evidence for a heightened interest in greenhouse-gas-free high-temperature processes, none of those have been implemented or demonstrated on industrial scale in the steam cracking process.

### Detailed Description

Steam cracking involves the exposure of a hydrocarbon feedstock (crude oil, gas oil, naphtha, ethane, propane, butane) to very high temperatures (750-1000°C) for a very short period of time (less than 1 second). During this very short period the feedstock is thermally decomposed into useful products such as ethylene, propylene, butadiene and benzene. Longer exposure times bring about undesirable consecutive reactions reducing the yield of useful products and causing fouling of the reactor.

In the hot zone as described previously, current reactor setups consist of narrow metal pipes where the hydrocarbon feedstock passes through at high speed, diluted with steam, where the pipes are externally heated by combustion of light hydrocarbons (often generated as byproducts of cracking) in a so-called firebox. The external flames heat the multiple metallic tubes by essentially radiation whereas the still hot flue gases, leaving the radiation zone, preheat the feedstock and dilution steam in the convection section through heat-exchange bundles.

As a matter of fact, the contact times and temperatures realized in the current equipment do not necessarily represent the most desirable combination of pressure, temperature and residence time from a perspective of product composition (which should be understood as total product value). Shorter residence times and higher temperatures are beyond reach given the speed of heat transfer. Higher temperatures require longer heat transfer and also to hotter tube walls, both leading to more side reactions. And shorter residence times require even higher heat transfer rates. Classic technology heats in an inhomogeneous way, producing hot spots that promote undesirable conversions.

A way to further improve the effectiveness of steam cracking is to apply different heat transfer techniques, either on their own or in combination. More specific, this is about heating technologies that do not rely on the transfer of heat from a hot metal surface to the hydrocarbon reaction mixture.

Apart from further optimization of current steam cracker practice, also another important driver for change has materialized, being the need to reduce greenhouse gas emissions in general, and the very high emissions from the heating of huge steam crackers in particular. With the primary supply of carbon neutral energy most likely being electrical in nature (electricity generated from nuclear, biomass, tidal & waves, geothermal, solar and wind sources) electrical techniques should be considered with priority.

One particular technique for this is direct heating of hydrocarbon feedstock molecules by a radiation induced plasma that interacts directly with the molecules. This has been described for microwave radiation (Fall et al, "Microwave Enhanced cracking of ethane to ethylene production", AICHE Spring National Meeting, Houston 2012). As a matter of fact, plasma conversion has been applied industrially for high-temperature processes for years (for a review, see Fincke et al, "plasma thermal conversion of methane to acetylene", Plasma Chemistry and plasma processing 22, 105-136 (2002). Some types of electromagnetic radiation (infrared, microwave, radiofrequency) show direct interaction with hydrocarbons, although no practical applications for high-temperature heating have been described. And electromagnetic fields changing with high frequency can heat up molecules through dipole interaction.

Another technique involves homogeneous heating of a gas by first accelerating a preheated gas to supersonic speeds, and subsequently sharply decelerating it, causing shockwaves. In the shockwaves near-instantaneous homogeneous heating to cracking temperatures occurs without the presence of even-hotter surfaces (which cause undesirable side reactions).

The principle of cracking by inducing shockwaves was described by van Goethem in 2010 in his thesis "Next Generation Steam Cracker Concept", where hydrocarbon feedstock is injected into a carrier gas moving at supersonic speeds, followed by deceleration. In this case, both the preheating and the acceleration methods are not specified and no reference is made to the potential of complete electrification (and consequent elimination of carbon emission).

Rockwell International Corp (US4724272) describes a shock wave reactor to pyrolyse methane. A fuel and oxidizer are combusted in a combustion zone to produce a hot gas stream at a superatmospheric pressure. The hot gas stream is then passed through a converging-diverging nozzle to accelerate the hot gas stream to a velocity of at least about mach 2. The reactant to be pyrolyzed is injected into the supersonic hot gas stream to produce a reaction mixture flowing at supersonic velocity and initiate the endothermic pyrolysis reactions. Substantially immediately thereafter the velocity of the reaction mixture is reduced over a predetermined reaction time to convert the kinetic energy of the reaction mixture to thermal energy in an amount sufficient to substantially offset the endothermic reactions taking place while maintaining supersonic flow. At the end of the predetermined reaction time the velocity of the reaction mixture is reduced to subsonic flow and the reaction quenched.

US5219530 (University of Washington) relates to an apparatus for initiating pyrolysis of a feedstock by establishing a continuous, standing shock wave. A shock wave reactor receives an ethane feedstock and a carrier fluid comprising superheated steam. The feedstock and the carrier fluid are pressurized so that they expand into parallel supersonic streams that mix due to turbulence within a mixing section of a longitudinally extending channel of the shock wave reactor. The pyrolysis vessel comprises a nozzle section disposed between the inlet and the outlet, which has a reduced cross-sectional area compared to that of adjacent portions of the pyrolysis vessel that receives the carrier fluid and a plurality of injection nozzles behind the reduced cross-sectional area that receives the feedstock. The carrier fluid heats the ethane feedstock as it mixes with it, producing a mixture that flows at supersonic velocity longitudinally down the channel. A gate valve disposed downstream of the channel provides a controlled back pressure that affects the position of the shock wave and the residence time for the reaction. The shock wave rapidly heats the mixture above a pyrolysis temperature, producing a desired product by cracking the feedstock. Rapid heating of the mixture enables a residence time in the pyrolysis section of only 5 to 50 milliseconds.

Further publications that describe heating of a cracking process by shockwaves are US5300216; US20160296904A1; US20180215615A1 and US10384180B2. All describe injection of a hydrocarbon feedstock into a hot carrier gas, which is often made by combustion of a feedstock.

In WO2019/221726A1 by Dresser Rand Company, a similar process and reactor configuration is described. In this case, a hydrocarbon gas is accelerated by an electrically driven compressor to supersonic speeds and then decelerated in a static diffuser, causing shockwaves, temperature spikes and cracking. The process is not completely electrified yet, this can be achieved by electrification of the preheating as described in this patent application.

Another variation has been provided by Finnish company Coolbrook, based on earlier Russian patents by Bushev (CN1298439A, CN102427875A). In this concept (rotodynamic) a hydrocarbon feedstock is accelerated and decelerated in a number of stages in a bladed reactor for the pyrolysis of hydrocarbons (EP2412430A4).

In all these propositions, the emphasis is on a combination of, in comparison with conventional steam cracking, a high yield of valuable products, especially ethylene, and a low production of undesirable side products such as coke. The possibilities to reduce greenhouse gas emissions by complete electrification is not mentioned.

Embodiments disclosed in the present application are directed to eliminating carbon dioxide emissions as much as possible from industrial steam cracking by applying shockwave technologies in the cracking section in combination with electrical preheating of the feedstock.

Embodiments of the present application thus provide a process for cracking a hydrocarbon feedstock according to claim 1.

Processes for applying a shockwave to the hydrocarbon feedstock are known as described above.

In subsonic aerodynamics, the phenomenon of flow heating by compression and dissipation is generally neglected. However, when supersonic speeds are reached in the flow, shock waves occur, associated notably with an increase of the temperature of the fluid. Such aerodynamic heating allows to add temperature to an electrically preheated reaction mixture at a temperature still below the incipient cracking temperature to rapidly reach the incipient cracking temperature by converting kinetic energy of the molecules into heat without requiring heat transfer through a reactor wall.

The physical mechanism behind aerodynamic heating with shock waves can be explained as follows (Anderson, John D. (2007). Fundamentals of Aerodynamics (4th ed.). McGraw-Hill.): the molecules at supersonic velocity that are decelerated in a diffuser section or by impacting bodies undergo a change in momentum; this change is transmitted to neighboring molecules at the local speed of sound. The upstream flow being supersonic, the disturbances (pressure pulses or sound waves) cannot propagate upstream and hence they pile up and coalesce, forming a standing wave. This shock wave is an extremely thin region at right angles to the flow direction of high gradients across which the flow properties change drastically. These abrupt, almost discontinuous variations are characterized by the Rankine-Hugoniot jump relations that explains the links between upstream and downstream velocity, pressure, temperature and density of the shock wave. The speed of sound in the fluid can be expressed as a = (γRT)^{0.5} where γ = Cp/Cv is the ratio of specific heats, R the gas constant and T the temperature.

The Mach number of the flow is defined as the ratio of local gas speed to the speed of sound and hence a flow is supersonic if its local gas speed is greater than its local speed of sound, or M > 1. The shock wave relations are obtained by applying the laws of conservation of mass, momentum, and energy from which three equations giving the density, velocity and temperature ratios, across a normal shock wave in terms of the pressure ratio across the shock wave can be derived. The pressure ratio is often termed the strength of the shock wave. The second law of thermodynamics requires that for an adiabatic process the entropy must remain unchanged or must increase, therefore follows that the shock wave must always be compressive, i.e. the pressure must always increase across the shock wave. Using the equations for the changes across a normal shock then shows that that the density always increases, the velocity always decreases, and the temperature always increases across a shock wave. These equations can also be expressed in terms of Mach numbers upstream and downstream of the shock wave. The conditions to reach a compressive shock wave (increase in entropy) demonstrates that the Mach number upstream must be supersonic (M>1) and the Mach number downstream has to be subsonic (M<1).

In the case of a normal shock wave, the velocities both upstream of the shock and downstream of the shock are at right angles to the shock wave. In the case of an oblique shock wave there is a change in flow direction across the shock. More complex shock wave may be effectively normal in part of the flow, curved in other parts of the flow and effectively oblique in other parts of the flow.Without willing to be bound to any theory, shock wave or aerodynamic heating can be accomplished by decelerating a supersonic flow to subsonic conditions by applying a diverting diffuser section, by placing blunt or concave bodies (wedges or cones) or combinations of the latter in a supersonic flow.

The present application further provides additional embodiments according to claims 2 to 14.

The feedstock for the present process may be ethane, liquefied petroleum gas, naphtha or gasoils or mixtures thereof. Liquefied petroleum gas (LPG) consists essentially of propane and butanes. Petroleum naphtha or naphtha is defined as the hydrocarbons fraction of petroleum having a boiling point from 15°C up to 200°C. It consists of a complex mixture of linear and branched paraffins (single and multi-branched), cyclic paraffins and aromatics having carbons numbers ranging from 5 to about 11 carbons atoms. Light naphtha has a boiling range from 15 to 90°C, consisting of C5 to C6 hydrocarbons while heavy naphtha has a boiling range from 90 to 200°C, consisting of C7 to about C11 hydrocarbons. Gasoils have a boiling range from about 200 to 350°C, consisting of C10 to C22 hydrocarbons, including essentially linear and branched paraffins, cyclic paraffins and aromatics (including mono-, naphtho- and poly-aromatic). Heavier gasoils (like atmospheric gasoil, vacuum gasoil, atmospheric residua and vacuum residua), having boiling ranges above 300°C and C20+ hydrocarbons including essentially linear and branched paraffins, cyclic paraffins and aromatics (including mono-, naphtho- and poly-aromatic) are available from atmospheric or vacuum distillations units.

In particular, the cracking products obtained in the present process may include ethylene, propylene and benzene, and optionally hydrogen, toluene, xylenes, and 1,3-butadiene.

In embodiments, the outlet temperature of the shock wave reactor may range from 820 to 1200°C, from 830 to 1100°C, from 840 to 950°C, or from 850°C to 940°C. The outlet temperature may influence the content of high value chemicals in the cracking products produced by the present process.

In embodiments, the residence time of the feedstock, through the section of the shock wave reactor where the temperature is between 650 and 1200°C, may range from 0.005 to 0.5 seconds, or from 0.01 to 0.4 seconds.

In embodiments, steam cracking said feedstock is done in presence of steam in a ratio of 0.1 to 1.0 kg steam per kg of hydrocarbon feedstock, from 0.25 to 0.7 kg steam per kg of hydrocarbon feedstock, or 0.35 kg steam per kg of feedstock mixture, to obtain cracking products as defined above.

In embodiments, the shock wave reactor outlet pressure may range from 500 to 1500 mbars, from 700 to 1000 mbars, or may be approx. 850 mbars. The residence time of the feed in the shock wave reactor and the temperature are to be considered together. A lower operating pressure may result in easier light olefins formation and reduced coke formation. The lowest pressure possible may be accomplished by (i) maintaining the output pressure of the shock wave reactor as close as possible to atmospheric pressure at the suction of the cracked gas compressor (ii) reducing the pressure of the hydrocarbons by dilution with steam (which has a substantial influence on slowing down coke formation). The steam/feedstock ratio may be maintained at a level sufficient to limit coke formation.

Effluent from the reactor may contain unreacted feedstock, desired olefins (mainly ethylene and propylene), hydrogen, methane, a mixture of C₄'s (primarily isobutylene and butadiene), pyrolysis gasoline (aromatics in the C₆ to C₈ range), ethane, propane, di-olefins (acetylene, methyl acetylene, propadiene), and heavier hydrocarbons that boil in the temperature range of fuel oil (pyrolysis fuel oil). This cracked gas may be rapidly quenched to 338-510°C to stop the pyrolysis reactions, minimize consecutive reactions and to recover the sensible heat in the gas by generating high-pressure steam in parallel transfer-line heat exchangers (TLE's). In gaseous feedstock-based plants, the TLE-quenched gas stream may flow forward to a direct water quench tower, where the gas may be cooled further with recirculating cold water. In liquid feedstock-based plants, a prefractionator may precede the water quench tower to condense and separate the fuel oil fraction from the cracked gas. In both types of plants, the major portions of the dilution steam and heavy gasoline in the cracked gas may be condensed in the water quench tower at 35-40°C. The water-quench gas may be subsequently compressed to about 25-35 Bars in 4 or 5 stages. Between compression stages, the condensed water and light gasoline may be removed, and the cracked gas may be washed with a caustic solution or with a regenerative amine solution, followed by a caustic solution, to remove acid gases (CO₂, H₂S and SO₂). The compressed cracked gas may be dried with a desiccant and cooled with propylene and ethylene refrigerants to cryogenic temperatures for the subsequent product fractionation: front-end demethanization, front-end depropanization or front-end deethanization.

The hydrocarbon feedstock and dilution steam are preheated before entering the shock wave reactor in several stages. First, most of the sensible and eventually the latent heat of the reactor effluent may be recovered by using heat exchangers while preheating the hydrocarbon feedstock and dilution steam or boiling feedwater used therefore.

Further preheating may be done by electric heating, either resistive, inductive, microwave or radiative heating.

Several physical concepts to heat a fluid by using electricity are known by the art (see Fundamentals of Electroheat Electrical Technologies for Process Heating, Springer International Publishing Switzerland 2017 and Industrial Process Heating: Current and Emerging Applications of Electrotechnologies, 2010 Electric Power Research Institute).

Resistive heating, also known as Joule heating or Ohmic heating, is the process by which the passage of an electric current through a resistor produces heat. Resistive heating has a coefficient of performance of 1.0, meaning that every joule of electrical energy supplied produces one joule of heat. The heated resistor transfers the heat to the hydrocarbon feedstock and dilution steam. In embodiments of the present application, this can be done in two ways. According to a first embodiment, pipes through which the hydrocarbon feedstock, dilution steam or combinations thereof flow, are resistors that heat up. According to a second embodiment, resistors in any shape (wires, spirals, rods etc) are placed inside tubular or duct envelopes through which the hydrocarbon feedstock, dilution steam or combinations thereof flow.

Induction heating is a non-contact method by which electrically conducting materials are heated in an alternating magnetic field. An electromagnetic field is produced by applying current with a given frequency to an inductor coil in proximity to the conductive workpiece. When the magnetic field intersects a workpiece made from any electrically conducting material, it generates a circulating current (called eddy currents), which generates heat. The heated conductor transfers the heat to the hydrocarbon feedstock and dilution steam. In embodiments of the present application, this can be done in two ways. According to a first embodiment, pipes through which the hydrocarbon feedstock, dilution steam or combinations thereof flow, are conductors that are heated up by the electromagnetic field. According to a second embodiment, conducting packing material in any shape (balls, extrudates, irregular particles etc) is placed inside tubular or duct envelopes through which the hydrocarbon feedstock, dilution steam or mixture thereof flows.

Microwave heating uses specific parts of the electromagnetic spectrum to internally heat low-conductive materials. Dielectric heating is accomplished with the application of electromagnetic fields. The material to be heated is placed between two electrodes that are connected to a high-frequency generator. The electromagnetic fields excite the molecular makeup of the material, thereby generating heat within the material: the microwaveable material must possess an asymmetric molecular structure, having electric dipoles. The electric dipoles try to align with the orientation of the electric field, generating movement of molecules, thereby generating heat. The heated material transfers the heat to the hydrocarbon feedstock and dilution steam. In embodiments of the present application, this can be done in two ways. According to a first embodiment, pipes through which the hydrocarbon feedstock, dilution steam or combinations thereof flow, are made from microwaveable material. According to a second embodiment, microwaveable packing material in any shape (balls, extrudates, irregular particles etc) are placed inside tubular or duct envelopes through which the hydrocarbon feedstock, dilution steam or combinations thereof flow inbetween the packing material.

Electric radiant heating uses heating elements that reach a high temperature that are sometimes enclosed by an envelope and equipped with reflectors to direct the energy output away from the body of the heater towards the receiving material. The element emits infrared radiation that travels through space until it hits an absorbing surface (like tubes that contain the feedstock, dilution steam or mixtures thereof), where it is partially converted to heat and partially reflected. In embodiments of the present application, this can be done by using pipes through which the hydrocarbon feedstock, dilution steam or combinations thereof flow, that are heated up by incident infrared radiation.

Renewable energy refers to energy from natural sources or processes that are constantly replenished on a human timescale, such as sunlight, wind, rain, tides, waves, hydropower and geothermal heat.

Low-carbon power refers to energy from processes or technologies that produce power with substantially lower amounts of carbon dioxide emissions than from conventional fossil fuel power generation. It includes low carbon power generation sources such as wind power, solar power, hydropower and nuclear power. The term largely excludes conventional fossil fuel sources.

The process(es) of the present application is/are particularly advantageous in that the electrically powered heat sources can use electricity originating from renewable or low-carbon energy. In this case, the process leads to less emission of CO₂ than a conventional steam cracking process. A possible definition of renewable or low-carbon electricity lies in the quantity of CO₂ that is emitted to produce it. Some general value of the quantity of CO₂ emitted during the production of electricity can be found in documents such as Koffi, Brigitte; Cerutti, Alessandro; Duerr, Marlene; lancu, Andreea; Kona, Albana; Janssens-Maenhout, Greet (2017): CoM Default Emission Factors for the Member States of the European Union - Version 2017, European Commission, Joint Research Centre (JRC) [Dataset]. Such document can be downloaded at: http://data.europa.eu/89h/jrc-com-ef-comw-ef-2017.

As a proposed definition of renewable or low-carbon electricity, it is considered that electricity produced with a standard emission factor of less than 200 kg (0.2 ton) CO₂ per MWh electricity is renewable or low-carbon electricity, preferably less than 100 kg (0.1 ton) CO₂/MWh or most preferably less than 50 kg (0.05 ton) CO₂/MWh. The standard emission factors are the emissions taking place due to consumption of energy carriers using the standard approach, i.e. by applying IPCC *"standard"* emission factors in line with IPCC principles for stationary combustion of the energy carriers. For clarity, other greenhouse gases like methane and nitrogen oxides are not accounted for in these standard emission factors. Also emissions related to the supply of the energy carriers are not accounted for in these emission factors as they can vary a lot according to regions and over time. By way of example, power generation from fossil resources emits the following amounts of CO₂: 320-330 kg CO₂/MWh emissions from the highest efficiency combined-cycle gas turbine (CCGT) plants, 500-520 kg CO₂/MWh for modern open-cycle gas turbine (OCGT) plants, and 750-800 kg CO₂/MWh for a modern supercritical coal-fired power plant whereas nuclear energy plants, geothermal, photovoltaic, hydropower and wind turbines emit 0 kg CO₂/MWh.

## Claims

1. A process for cracking a hydrocarbon feedstock, comprising the steps of:
• feeding hydrocarbon feedstock and dilution steam to a reactor;
• bringing the hydrocarbon feedstock and dilution steam to supersonic velocities in the reactor at a temperature T1;
• adding further dilution steam at a temperature T2, where T2 is higher than T1; followed by
• applying a shockwave to the preheated hydrocarbon feedstock and dilution steam mixture to induce cracking of the hydrocarbon feedstock, to convert at least a part of the hydrocarbon mixture to produce olefins;
wherein the hydrocarbon feedstock, the dilution steam and/or a mixture thereof are preheated before entering the reactor and wherein the preheating is carried out at least partially by means of one or more electrically powered heat sources,
wherein sensible or latent heat of effluent exiting the reactor is extracted at least partially by means of one or more heat exchangers and used to at least partially preheat the hydrocarbon feedstock, dilution steam or mixture thereof,
wherein the electrically powered heat sources are powered by electricity produced with a standard emission factor of less than 200 kg (0.2 ton) CO₂ per MWh electricity.

2. The process according to claim 1, wherein the diluted steam to hydrocarbon weight ratio in the reactor is between 0.1 and 1.0 (wt./wt.) and
wherein the hydrocarbon and dilution steam mixture has a temperature less than 800°C before reaching supersonic velocities.

3. The process of claim 1 or 2, wherein the hydrocarbon feedstock comprises one of more hydrocarbons having at least two carbon atoms, in particular selected from the group consisting of ethane, liquefied petroleum gas, naphtha and gasoils.

4. The process according to any one of the preceding claims, wherein the preheating is done by resistive heating of at least one electrically conductive pipe containing the hydrocarbon feedstock, dilution steam or mixture thereof,

5. The process according to any one of the preceding claims, wherein the preheating is done by resistive heating of electrically conductive elements within at least one pipe containing the hydrocarbon feedstock, dilution steam or mixture thereof.

6. The process according to any one of the preceding claims, wherein the preheating is done by inductive heating of at least one electrically conductive pipe containing the hydrocarbon feedstock, dilution steam or mixture thereof.

7. The process according to any one of the preceding claims, wherein the preheating is done by inductive heating of electrically conductive packing material within at least one pipe containing the hydrocarbon feedstock, dilution steam or mixture thereof.

8. The process according to any one of the preceding claims, wherein the preheating is done by heating at least one pipe containing the hydrocarbon feedstock, dilution steam or mixture thereof by an electrically powered external source of infrared radiation.

9. The process according to any one of the preceding claims, wherein the preheating is done by microwave heating of at least one electrically low-conductive pipe containing the hydrocarbon feedstock, dilution steam or mixture thereof.

10. The process according to any one of the preceding claims, wherein the preheating is done by microwave heating of electrically low-conductive packing material within at least one pipe containing the hydrocarbon feedstock, dilution steam or mixture thereof.

11. The process according to any one of the preceding claims, wherein the preheating is done by heating of packing materials within at least one pipe containing hydrocarbon feedstock, dilution steam or mixture thereof by microwave interaction, preferably with silicon carbide.

12. The process according to any one of the preceding claims, wherein the preheating of the hydrocarbon feedstock and of the dilution steam is carried out separately in at least two pipes before mixing the two streams; or
wherein the hydrocarbon feedstock and the dilution steam are mixed, followed by simultaneous preheating the mixture; or
wherein the preheating of the hydrocarbon feedstock and the dilution steam is carried out separately in at least two pipes to a temperature less than 500°C, followed by mixing the two streams and further preheated the mixture simultaneously.

13. The process according to any one of the preceding claims, wherein the supersonic velocities are applied by means of an electric driven impeller.

14. The process according to any one of the preceding claims, comprising
• recovering a methane stream containing essentially methane produced during cracking of the hydrocarbon feedstock;
• feeding the methane stream and dilution steam to a reactor;
• bringing the methane stream and dilution steam to supersonic velocities in the reactor, followed by applying a shockwave to induce pyrolysis of the methane, to convert at least a part of the methane to produce hydrogen, acetylenes and olefins;
• mixing the products of reactor effluent with the products of the reactor effluent of the cracking of the hydrocarbon feedstock.
wherein the methane stream, the dilution steam and/or a mixture thereof are preheated before entering the reactor and wherein the preheating is carried out at least partially by means of one or more electrically powered heat sources.

## Patentansprüche

1. Verfahren zum Cracken eines Kohlenwasserstoff-Beschickungsmaterials, das folgende Schritte umfasst:
• Beschicken eines Reaktors mit Kohlenwasserstoff-Beschickungsmaterial und Verdünnungsdampf;
• Bringen des Kohlenstoff-Beschickungsmaterials und des Verdünnungsdampfs auf Überschallgeschwindigkeit im Reaktor bei einer Temperatur T1;
• Zugeben von weiterem Verdünnungsdampf bei einer Temperatur T2, wobei T2 höher ist als T1; darauf folgend
• Einleiten einer Druckwelle in die vorerwärmte Mischung aus Kohlenwasserstoff-Beschickungsmaterial und Verdünnungsdampf zum Bewirken des Crackens des Kohlenwasserstoff-Beschickungsmaterials und damit Umwandeln von zumindest einem Teil der Kohlenwasserstoffmischung zum Erzeugen von Olefinen;
wobei das Kohlenwasserstoff-Beschickungsmaterial, der Verdünnungsdampf und/oder eine Mischung daraus vor dem Einleiten in den Reaktor vorerwärmt werden, und wobei das Vorerwärmen zumindest teilweise mit einer oder mehreren elektrisch betriebenen Wärmequellen erfolgt,
wobei sensible oder latente Wärme von aus dem Reaktor ausströmendem Austrag zumindest teilweise mit einem oder mehreren Wärmetauschern entnommen und dafür eingesetzt wird, das Kohlenwasserstoff-Beschickungsmaterial, den Verdünnungsdampf oder die Mischung daraus zumindest teilweise vorzuwärmen,
wobei die elektrisch betriebenen Wärmequellen mit elektrischer Energie betrieben werden, die mit einem Standardemissionsfaktor von unter 200 kg (0,2 Tonne) CO₂ pro MWh elektrischer Energie erzeugt wird.

2. Verfahren nach Anspruch 1, wobei das Gewichtsverhältnis von verdünntem Dampf zu Kohlenwasserstoffen im Reaktor zwischen 0,1 und 1,0 (Gew./Gew.) beträgt und
wobei die Mischung aus Kohlenwasserstoffen und Verdünnungsdampf vor dem Erreichen der Überschallgeschwindigkeit eine Temperatur von unter 800°C aufweist.

3. Verfahren nach Anspruch 1 oder 2, wobei das Kohlenstoff-Beschickungsmaterial einen oder mehrere Kohlenwasserstoffe mit mindestens zwei Kohlenstoffatomen umfasst, die insbesondere aus der Gruppe ausgewählt sind, die aus Ethan, Flüssiggas, Naphtha und Dieselölen besteht.

4. Verfahren nach einem der vorhergehenden Ansprüche, wobei das Vorerwärmen durch Widerstandbeheizen von mindestens einem elektrisch leitfähigen Rohr erfolgt, das das Kohlenwasserstoff-Beschickungsmaterial, den Verdünnungsdampf oder die Mischung daraus enthält.

5. Verfahren nach einem der vorhergehenden Ansprüche, wobei das Vorerwärmen durch Widerstandbeheizen von elektrisch leitfähigen Elementen in mindestens einem Rohr erfolgt, das das Kohlenwasserstoff-Beschickungsmaterial, den Verdünnungsdampf oder die Mischung daraus enthält.

6. Verfahren nach einem der vorhergehenden Ansprüche, wobei das Vorerwärmen durch Induktionserwärmen von mindestens einem elektrisch leitfähigen Rohr erfolgt, das das Kohlenwasserstoff-Beschickungsmaterial, den Verdünnungsdampf oder die Mischung daraus enthält.

7. Verfahren nach einem der vorhergehenden Ansprüche, wobei das Vorerwärmen durch Induktionserwärmen von elektrisch leitfähigem Packungsmaterial in mindestens einem Rohr erfolgt, das das Kohlenwasserstoff-Beschickungsmaterial, den Verdünnungsdampf oder die Mischung daraus enthält.

8. Verfahren nach einem der vorhergehenden Ansprüche, wobei das Vorerwärmen durch Erwärmen von mindestens einem Rohr, das das Kohlenwasserstoff-Beschickungsmaterial, den Verdünnungsdampf oder die Mischung daraus enthält, mit einer elektrisch betriebenen externen Infrarotquelle erfolgt.

9. Verfahren nach einem der vorhergehenden Ansprüche, wobei das Vorerwärmen durch Erwärmen von mindestens einem Rohr mit geringer elektrischer Leitfähigkeit mit Mikrowellen erfolgt, das das Kohlenwasserstoff-Beschickungsmaterial, den Verdünnungsdampf oder die Mischung daraus enthält.

10. Verfahren nach einem der vorhergehenden Ansprüche, wobei das Vorerwärmen durch Erwärmen von Packungsmaterial mit geringer elektrischer Leitfähigkeit in mindestens einem Rohr mit Mikrowellen erfolgt, das das Kohlenwasserstoff-Beschickungsmaterial, den Verdünnungsdampf oder die Mischung daraus enthält.

11. Verfahren nach einem der vorhergehenden Ansprüche, wobei das Vorerwärmen durch Erwärmen von Packungsmaterial in mindestens einem Rohr, das das Kohlenwasserstoff-Beschickungsmaterial, den Verdünnungsdampf oder die Mischung daraus enthält, durch Wechselwirkung von Mikrowellen, vorzugsweise mit Siliziumcarbid, erfolgt.

12. Verfahren nach einem der vorhergehenden Ansprüche, wobei das Vorerwärmen des Kohlenwasserstoff-Beschickungsmaterials und des Verdünnungsdampfs separat in mindestens zwei Rohren erfolgt, bevor die beiden Ströme gemischt werden, oder
wobei das Kohlenwasserstoff-Beschickungsmaterial und der Verdünnungsdampf gemischt werden, darauf gleichzeitig das Vorerwärmen der Mischung, oder
wobei das Vorerwärmen des Kohlenwasserstoff-Beschickungsmaterials und des Verdünnungsdampfs separat in mindestens zwei Rohren auf eine Temperatur von unter 500°C erfolgt, darauf gleichzeitig ein Mischen der beiden Ströme und ein weiteres Vorerwärmen der Mischung.

13. Verfahren nach einem der vorhergehenden Ansprüche, wobei die Überschallgeschwindigkeit mit einem elektrisch betriebenen Laufrad aufgebracht wird.

14. Verfahren nach einem der vorhergehenden Ansprüche, umfassend:
• Gewinnen eines Methanstroms, der im Wesentlichen Methan enthält und während des Crackens des Kohlenwasserstoff-Beschickungsmaterials erzeugt wurde;
• Beschicken eines Reaktors mit dem Methanstrom und Verdünnungsdampf;
• im Reaktor Bringen des Methanstroms und des Verdünnungsdampfs auf Überschallgeschwindigkeit, darauf Einleiten einer Druckwelle zum Bewirken einer Pyrolyse des Methans und damit Umwandeln von zumindest einem Teil des Methans zum Erzeugen von Wasserstoff, Acetylenen und Olefinen;
• Mischen der Produkte des Reaktoraustrags mit den Produkten des Reaktoraustrags vom Cracken des Kohlenwasserstoff-Beschickungsmaterials,
wobei der Methanstrom, der Verdünnungsdampf und/oder eine Mischung daraus vor dem Einleiten in den Reaktor vorerwärmt werden, und wobei das Vorerwärmen zumindest teilweise mit einer oder mehreren elektrisch betriebenen Wärmequellen erfolgt.

## Revendications

1. Procédé de craquage d'une charge d'hydrocarbures, comprenant les étapes de :
• introduction d'une charge d'hydrocarbures et de vapeur de dilution dans un réacteur ;
• soumission de la charge d'hydrocarbures et de la vapeur de dilution à des vitesses supersoniques dans le réacteur, à une température T1 ;
• ajout de vapeur de dilution supplémentaire à une température T2, où T2 est supérieure à T1 ; puis
• application d'une onde de choc au mélange préchauffé de charge d'hydrocarbures et de vapeur de dilution pour induire le craquage de la charge d'hydrocarbures, afin de transformer au moins une partie du mélange d'hydrocarbures pour produire des oléfines ;
dans lequel la charge d'hydrocarbures, la vapeur de dilution et/ou leur mélange sont préchauffés avant d'entrer dans le réacteur, et dans lequel le préchauffage est effectué au moins partiellement au moyen d'une ou plusieurs sources de chaleur alimentées électriquement,
dans lequel la chaleur sensible ou latente d'un effluent sortant du réacteur est extraite au moins partiellement au moyen d'un ou plusieurs échangeurs de chaleur et est utilisée pour préchauffer au moins partiellement la charge d'hydrocarbures, la vapeur de dilution ou leur mélange,
dans lequel les sources de chaleur alimentées électriquement sont alimentées par de l'électricité produite avec un facteur d'émission standard inférieur à 200 kg (0,2 tonne) de CO₂ par MWh d'électricité.

2. Procédé selon la revendication 1, dans lequel le rapport pondéral entre la vapeur diluée et les hydrocarbures dans le réacteur est compris entre 0,1 et 1,0 (poids/poids) et dans lequel le mélange d'hydrocarbures et de vapeur de dilution a une température inférieure à 800°C avant d'atteindre les vitesses supersoniques.

3. Procédé de la revendication 1 ou 2, dans lequel la charge d'hydrocarbures comprend un ou plusieurs hydrocarbures ayant au moins deux atomes de carbone, en particulier choisis dans le groupe constitué par l'éthane, le gaz de pétrole liquéfié, le naphta et les gazoles.

4. Procédé selon l'une quelconque des revendications précédentes, dans lequel le préchauffage est réalisé par chauffage résistif d'au moins une conduite électriquement conductrice contenant la charge d'hydrocarbures, la vapeur de dilution ou leur mélange.

5. Procédé selon l'une quelconque des revendications précédentes, dans lequel le préchauffage est réalisé par chauffage résistif d'éléments électriquement conducteurs à l'intérieur d'au moins une conduite contenant la charge d'hydrocarbures, la vapeur de dilution ou leur mélange.

6. Procédé selon l'une quelconque des revendications précédentes, dans lequel le préchauffage est réalisé par chauffage inductif d'au moins une conduite électriquement conductrice contenant la charge d'hydrocarbures, la vapeur de dilution ou leur mélange.

7. Procédé selon l'une quelconque des revendications précédentes, dans lequel le préchauffage est réalisé par chauffage inductif d'un matériau de remplissage électriquement conducteur à l'intérieur d'au moins une conduite contenant la charge d'hydrocarbures, la vapeur de dilution ou leur mélange.

8. Procédé selon l'une quelconque des revendications précédentes, dans lequel le préchauffage est réalisé en chauffant au moins une conduite contenant la charge d'hydrocarbures, la vapeur de dilution ou leur mélange, par une source externe de rayonnement infrarouge alimentée électriquement.

9. Procédé selon l'une quelconque des revendications précédentes, dans lequel le préchauffage est réalisé par chauffage par micro-ondes d'au moins une conduite électriquement peu conductrice contenant la charge d'hydrocarbures, la vapeur de dilution ou leur mélange.

10. Procédé selon l'une quelconque des revendications précédentes, dans lequel le préchauffage est réalisé par chauffage par micro-ondes d'un matériau de remplissage électriquement peu conducteur à l'intérieur d'au moins une conduite contenant la charge d'hydrocarbures, la vapeur de dilution ou leur mélange.

11. Procédé selon l'une quelconque des revendications précédentes, dans lequel le préchauffage est réalisé par chauffage de matériaux de remplissage à l'intérieur d'au moins une conduite contenant la charge d'hydrocarbures, la vapeur de dilution ou leur mélange, par interaction de micro-ondes, de préférence avec du carbure de silicium.

12. Procédé selon l'une quelconque des revendications précédentes, dans lequel le préchauffage de la charge d'hydrocarbures et de la vapeur de dilution est effectué séparément dans au moins deux conduites avant de mélanger les deux flux ; ou
dans lequel la charge d'hydrocarbures et la vapeur de dilution sont mélangées, et s'ensuit un préchauffage simultané du mélange ; ou
dans lequel le préchauffage de la charge d'hydrocarbures et de la vapeur de dilution est effectué séparément dans au moins deux conduites à une température inférieure à 500°C, et s'ensuit le mélange des deux flux et un préchauffage supplémentaire simultané du mélange.

13. Procédé selon l'une quelconque des revendications précédentes, dans lequel les vitesses supersoniques sont appliquées au moyen d'une roue à entraînement électrique.

14. Procédé selon l'une quelconque des revendications précédentes, comprenant
• la récupération d'un flux de méthane contenant essentiellement du méthane produit lors du craquage de la charge d'hydrocarbures ;
• l'introduction du flux de méthane et de vapeur de dilution dans un réacteur ;
• la soumission du flux de méthane et de la vapeur de dilution à des vitesses supersoniques dans le réacteur, suivie de l'application d'une onde de choc pour induire la pyrolyse du méthane, afin de transformer au moins une partie du méthane pour produire de l'hydrogène, des acétylènes et des oléfines ; et
• le mélange des produits de l'effluent du réacteur avec les produits de l'effluent du réacteur du craquage de la charge d'hydrocarbures ;
dans lequel le flux de méthane, la vapeur de dilution et/ou leur mélange sont préchauffés avant d'entrer dans le réacteur, et dans lequel le préchauffage est effectué au moins partiellement au moyen d'une ou plusieurs sources de chaleur alimentées électriquement.
